Europäisches Patentamt

European Patent Office

Office européen des brevets

(11) Numéro de publication : **0 003 855**
**B1**

(12) **FASCICULE DE BREVET EUROPÉEN**

(45) Date de publication du fascicule du brevet :
30.03.83

(21) Numéro de dépôt : 79200064.8

(22) Date de dépôt : 07.02.79

(51) Int. Cl.³ : **A 61 K   7/16**

(54) **Pâte dentifrice permettant un contrôle du temps de brossage des dents.**

(30) Priorité : 16.02.78 CH 1690/78

(43) Date de publication de la demande :
05.09.79 Bulletin 79/18

(45) Mention de la délivrance du brevet :
30.03.83 Bulletin 83/13

(84) Etats contractants désignés :
**BE DE FR GB IT LU NL SE**

(56) Documents cités :
DE A 1 767 662
FR A 902 143
GB A 29 565

**CHEMICAL ABSTRACTS**, Vol. 82, no. 4, 27 janvier
1975 Columbus Ohio, USA, **ABE et al. : « Color
indicators for mouth cleaning agents »**, abrégé
21737d

(73) Titulaire : **Blanc, Bernard
Chemin de Champagne, 18
CH-1025 Saint-Sulpice (CH)**

(72) Inventeur : **Monnerat, Bernard
CH-1701 Fribourg-Moncor (CH)**
Inventeur : **Assal, Jacques
Avenue du Théâtre, 7
CH-1005 Lausanne (CH)**
Inventeur : **Blanc, Bernard
Chemin de Champagne, 18
CH-1025 St-Sulpice (CH)**

(74) Mandataire : **Meylan, Robert Maurice
c/o BUGNION S.A. 10, route de Florissant Case
Postale 375
CH-1211 Genève 12-Champel (CH)**

Jouve, 18, rue St-Denis, 75001 Paris, France

## 0 003 855

### Pâte dentifrice permettant un contrôle du temps de brossage des dents

La présente invention concerne une pâte dentifrice qui contient des substances permettant de contrôler le temps de brossage des dents, par le changement de couleur qui se produit après un temps déterminé de brossage.

Les recherches les plus récentes en odontologie font apparaître le rôle prépondérant du temps de brossage des dents, alors que le rôle chimique de la pâte dentifrice n'aurait qu'une importance secondaire. La conclusion est que l'action de nettoyage mécanique des dents et de massage des gencives est d'une importance fondamentale ; elle doit être énergique et durer au moins une à deux minutes. Le brossage des dents étant une opération fastidieuse, la plupart des personnes, surtout les enfants, la bâclent très souvent en pensant à autre chose, et le contrôle de sa durée est illusoire.

On a proposé diverses pâtes dentifrices permettant un contrôle du temps de brossage par le fait qu'elles contiennent un réactif susceptible de virer de couleur par dilution ou modification du pH.

On a ainsi proposé d'additionner au dentifrice un indicateur de l'acidité du magma recouvrant les dents ou d'un indicateur d'acidité virant à un pH sensiblement égal à celui de la salive, mais sans donner de formules complètes et pratiquement utilisables.

Le DE-A-1 767 662 décrit une pâte dentifrice contenant des substances réactives pour le contrôle du temps de brossage des dents. Les substances réactives comprennent essentiellement un tampon, par exemple à base d'acide citrique, et un indicateur de pH tel que le phénolphtaléine ou le rouge de méthyle.

L'utilisation des pâtes dentifrices contenant l'un ou l'autre des indicateurs précités présente quelques inconvénients. En effet aussi bien le phénolphtaléine que le rouge de méthyle sont presque insolubles à l'eau et un rinçage très soigneux est nécessaire pour enlever les restes de la pâte colorée. En plus, le rouge de méthyle est instable car il est facilement réductible tandis que le phénolphtaléine est sensible au dioxyde de carbone.

Le brevet FR-A-902 143 décrit un produit destiné à détartrer et polir les dents par l'emploi de deux gels successifs. Le premier étant acide et le second basique. Le premier gel contient comme indicateur le rouge de tournesol qui change de couleur après l'application du second gel et un brossage attentif. Ce dernier produit est destiné à être utilisé surtout par un spécialiste par exemple un médecin-dentiste d'ailleurs le virage de la couleur du rouge de tournesol est faible, donc, difficilement observable par un utilisateur non initié. Le virage de couleur du rouge de tournesol se situe dans une large gamme de pH comprise entre 4 et plus 8 rendant ainsi l'observation aléatoire car cette dernière gamme ne se retrouve pas dans la salive.

Enfin les pigments de xanthène proposés comme indicateur dans une pâte dentifrice (Chemical Abstracts, vol. 82, 21737d) semblent être des pigments colorés enveloppés d'une matière soluble qui fond peu à peu durant le brossage de dents libérant ainsi les pigments indépendamment du pH de la salive.

D'autre part dans le choix des constituants, il y a lieu de tenir compte de la nocivité, même minime des réactifs, car les prescriptions en matière de toxicité deviennent de plus en plus sévères.

La présente invention apporte une solution pratique à ces problèmes en ne proposant qu'un indicateur non nocif et admis dans les produits alimentaires, facilement soluble dans l'eau et stable chimiquement malgré que sa structure chimique est similaire à celle du phénolphtaléine. La pâte dentifrice sera colorée par l'indicateur dont la teinte qu'il présente dans le domaine acide, au début du brossage, vire à une teinte différente lorsqu'il atteint le pH de la salive, à la fin du brossage.

La pâte dentifrice selon la présente invention, est caractérisée par le fait que les substances réactives comprennent essentiellement un tampon citrate/acide citrique, de molarité comprise entre 0,07 et 0,35 M avec un pH compris entre 4,0 et 5,5 au dosage de 10 à 50 %, et du rouge de chlorophénol au dosage de 0,02 à 0,1 %.

La formule la plus simple est de prendre comme matière de base une pâte dentifrice usuelle, mais ne présentant pas de réaction alcaline. Elle peut contenir des produits fluorés et médicaux.

Dans cette pâte, on introduit un tampon citrate/acide citrique de molarité comprise entre 0,07 et 0,35 M, et de pH compris entre 4,0 et 5,5 au dosage de 10 à 50 %, puis on y ajoute du rouge de chlorophénol au dosage de 0,02 à 0,1 %.

Le rouge de chlorophénol a été choisi parce que cet indicateur coloré de pH n'est ni toxique, ni cancérigène. Il ne tache pas les dents, ni les obturations, ni les prothèses. Sa teinte dans le domaine acide est jaune, et· dans le domaine du pH de la salive, il vire au pourpre. En plus malgré que sa structure chimique est similaire à celle du phénolphtaléine il est facilement soluble dans l'eau et il n'est pas sensible chimiquement aux différents composants de l'atmosphère dans laquelle il est utilisé. Ces effets surprenants et non prévisibles sont peut être justifiés par le fait que le groupe phtalide du phénolphtaléine a été substitué par le groupe sulfone dans le rouge du chlorophénol. Le groupe sulfone étant connu dans la fabrication des détergents comme provoquant une certaine lyophilie.

Si dans cette première formule, le pH final est ajusté à env. 4,5, le pouvoir tampon (exprimé dans ce cas en ml NaOH 0,1 N/g de pâte, utilisé en titration électrométrique jusqu'à pH 7) sera normalement compris entre 1,5 et 1,8 et le temps de brossage des dents, pour des personnes ayant un pH de salive entre 6 et 7, sera de 1 minute 15 secondes à 1 minute 45 secondes, selon le pH et la production de salive individuels.

# 0 003 855

Pour éviter une certaine sensation d'acidité qui peut apparaître lors de l'utilisation du dentifrice selon cette première formule, une deuxième formule a été mise au point, qui présente de plus l'avantage d'une meilleure stabilité du pH.

Cette deuxième formule se compose de :

— mélange de polymères synthétiques (carboxypolyméthylènes) et de celluloses modifiées, maximum 3,5 % ;
— acide silicique : 8 à 25 % ;
— silice : 1 à 6 % ;
— dodécylhydrogensulfate de sodium : 1,5 à 3 % ;
— propandiol : 3 à 4 % ;
— glycérine ou sorbitol : 20 à 60 % ;
— esters de l'acide p-hydroxybenzoïque : 0,1 à 0,5 % ;
— rouge de chlorophénol : 0,02 à 0,1 % ;
— colorants alimentaires : 0,01 à 0,04 % ;
— arômes : environ 1,5 % ;
— tampon citrate/acide citrique de force ionique et de pH adaptés pour obtenir un pH final compris entre 4,0 et 5,5 : 10 à 50 % ;
— eau ad 100 %.

Pour préciser davantage cette deuxième formule, disons que :

— les polymères synthétiques (carboxypolyméthylène) et les celluloses modifiées (carraghenate de sodium) seront avantageusement un mélange en parties sensiblement égales de Carbopol 940 et de Satiagum B ;
— les esters de l'acide benzoïque sont un mélange en parties sensiblement égales de p-hydroxybenzoate de méthyle et de p-hydroxybenzoate de propyle ;
— le tampon citrate/acide citrique sera avantageusement constitué d'acide citrique 0,07 M et de citrate de sodium 0,07 M ajusté à un pH de 4,2 à 4,6 ;
— les arômes peuvent être un mélange d'essences de menthe, d'anis, de cannelle, et d'eugénol ou autres.

Remarquons que, pour des raisons commerciales, la teinte jaune de la pâte dentifrice étant peu engageante, on peut ajouter un colorant alimentaire de couleur rouge ou orange, le rouge ponceau par exemple, en concentration faible, de façon à ne pas gêner l'apparition de la couleur de virage, et ainsi donner une teinte de départ rosée plus agréable.

Différentes variantes de cette formule générale sont évidemment possibles. On peut y incorporer un composé fluoré et un antiseptique. La formule suivante est un exemple d'exécution préférée :

— carboxypolyméthylène (Carbopol 940) : 1,5 % ;
— carraghénate de sodium (Satiagum B) : 1,5 % ;
— acide silicique : 10,0 % ;
— dioxyde de titane : 4,0 % ;
— laurylsulfate de sodium : 2,1 % ;
— glycérine ou sorbitol à 70 % : 33,0 % ;
— p-hydroxybenzoate de méthyle : 0,15 % ;
— p-hydroxybenzoate de propyle : 0,15 % ;
— rouge de chlorophénol : 0,05 % ;
— arômes : 1,3 % ;
— saccharine sodique : 0,5% ;
— monofluorophosphate de sodium : 0,75 % ;
— bromochlorophène : 0,15 % ;
— tampon pH 4,5 ad 100,0 g.

La pâte dentifrice selon cette formule préférée possède un pouvoir tampon compris entre 1,6 et 2,0 et le temps de brossage jusqu'au virage au pourpre est de 1 minute 30 secondes à 2 minutes.

Un simple rinçage de la bouche élimine toute coloration.

## Revendications

1. Pâte dentifrice contenant des substances réactives permettant de contrôler le temps de brossage des dents par le changement de couleur qui se produit après un temps déterminé de brossage, caractérisée par le fait que les substances réactives comprennent essentiellement un tampon citrate/acide citrique de molarité comprise entre 0,07 et 0,35 M avec un pH compris entre 4 et 5,5 au

3

dosage de 10 à 50 % et du rouge de chlorophénol au dosage de 0,02 à 0,1 %.

2. Pâte dentifrice selon la revendication 1, caractérisée par le fait qu'elle contient :

— mélange de polymères synthétiques (carboxypolyméthylènes) et de celluloses modifiées (carraghénate de sodium) : maximum 3,5 % ;
— acide silicique : 8-25 % ;
— silice : 1-6 % ;
— dodécylhydrogensulfate de sodium : 1,5 à 3 % ;
— propandiol : 3 à 4 % ;
— sorbitol : 20 à 60 % ;
— esters de l'acide p-hydroxybenzoïque : 0,1 à 0,5 % ;
— rouge de chlorophénol : 0,02 à 0,1 % ;
— arômes : env. 1,5 % ;
— colorants alimentaires : 0,01 à 0,04 % ;
— tampon citrate/acide citrique de force ionique et de pH adaptés pour obtenir un pH final compris entre 4,0 et 5,5 : 10 à 50 % ;
— eau ad 100 %.

3. Pâte dentifrice selon la revendication 2, caractérisée par le fait que le carboxypolyméthylène et le carraghénate sont le Carbopol 940 et le Satiagum B.

4. Pâte dentifrice selon la revendication 2, caractérisée par le fait que les esters de l'acide p-hydroxybenzoïque sont un mélange de p-hydroxybenzoate de méthyle et de p-hydroxybenzoate de propyle.

5. Pâte dentifrice selon la revendication 2, caractérisée par le fait que le tampon citrate est constitué d'acide citrique de 0,07 M et de citrate de sodium de 0,07 M, ajusté à un pH compris entre 4,2 et 4,6.

6. Pâte dentifrice selon la revendication 2, caractérisée par le fait que le colorant alimentaire est le rouge ponceau, en concentration faible (0,01 à 0,04 %), de façon à ne pas gêner l'apparition de la couleur de virage au pourpre, du rouge de chlorophénol.

7. Pâte dentifrice selon la revendication 1, caractérisée par le fait qu'elle contient, selon un exemple d'exécution préférée :

| | |
|---|---|
| — carbopol 940 | 1,5 % |
| — satiagum B | 1,5 % |
| — acide silicique | 10,0 % |
| — dioxyde de titane | 4,0 % |
| — laurysulfate de sodium | 2,1 % |
| — sorbitol 70 % | 33,0 % |
| — p-hydroxybenzoate de méthyle | 0,15 % |
| — p-hydroxybenzoate de propyle | 0,15 % |
| — rouge de chlorophénol | 0,05 % |
| — arômes | 1,3 % |
| — saccharine sodique | 0,5 % |
| — monofluorophosphate de sodium | 0,75 % |
| — bromochlorophène | 0,15 % |
| — tampon : acide citrique/citrate de sodium 0,07 M, pH 4,5 ad | 100,00 g |

**Claims**

1. A toothpaste containing reagents for controlling the tooth brushing time by the change in color occurring after a predetermining brushing time, characterised in that the reagents comprise essentially a citrate-citric acid buffer having a molarity within the range of 0.07 to 0.35 M with a pH of 4 to 5.5 in the proportion of 10 to 50 % and chlorophenol red in the proportion of 0.02 to 0.1 %.

2. A toothpaste according to claim 1, characterised in that it contains

— a mixture, not in excess of 3.5 % of synthetic polymers (carboxypolymethylenes) and modified celluloses (sodium carragheenate) ;
— 8 to 25 % silicic acid ;
— 1 to 6 % silica ;
— 1.5 to 3 % Sodium dodecylhydrogensulphate ;
— 3 to 4 propandiol ;
— 20 to 60 % sorbitol ;
— 0.1 to 0.5 % p-hydroxybenzoic acid esters ;
— 0.02 to 0.1 % chlorophenol red ;
— about 1.5 % aromas ;

4

— 0.01 to 0.04 % foodstuff dyes ;

— 10 to 50 % of a citrate/citric acid buffer having an ionic strength and a pH value adapted to yield a final pH within the range of 4.0 to 5.5 ;

— water, q. s. 100 %.

3. Toothpaste according to claim 2, in which the carboxypolymethylene and carragheenate consist of Carbopol 940 and Satiagum B.

4. A toothpaste according to claim 2, in which said esters of p-hydroxybenzoic acid are a mixture of methyl p-hydroxybenzoate and propyl p-hydroxybenzoate.

5. A toothpaste according to claim 2, in which said citrate buffer consists of 0.07 M citric acid and 0.07 M sodium citrate set at a pH of 4.2 to 4.6.

6. Toothpaste according to claim 2, in which said foodstuff dye is a moderately concentrated corn-poppy red (0.01 to 0.04 %) in order not to interfere with the turning of the chlorophenol red to purple red.

7. Toothpaste according to claim 1, in which the preferred composition is :

| | |
|---|---|
| — Carbopol 940 | 1.5 % |
| — Satiagum B | 1.5 % |
| — Silicic acid | 10.0 % |
| — Titanium dioxide | 4.0 % |
| — Sodium laurylsulphate | 2.1 % |
| — 70 % Sorbitol | 33.0 % |
| — Methyl p-hydroxybenzoate | 0.15 % |
| — Propyl p-hydroxybenzoate | 0.15 % |
| — Chlorophenol red | 0.05 % |
| — Aromas | 1.3 % |
| — Sodium Saccharin | 0.5 % |
| — Sodium monofluorophosphate | 0.75 % |
| — Bromochlorophene | 0.15 % |
| — Buffer : 0.07 M citric acid/sodium citrate, pH = 4.5   q. s. | 100.00 g |

## Ansprüche

1. Zahnpaste mit Reagenzien zur Kontrolle der Dauer des Zahnputzvorganges durch Farbänderung, die nach einer bestimmten Zeit des Putzens auftritt, dadurch gekennzeichnet, dass diese Reagenzien im wesentlichen einen Zitrat-/Zitronensäure-Puffer mit einer Molarität zwischen 0,07 und 0,35 M und einen pH zwischen 4 und 5,5 in einem Anteil von 10 bis 50 % sowie Chlorphenolrot in einem Anteil von 0,02 bis 0,1 % enthalten.

2. Zahnspaste nach Anspruch 1, dadurch gekennzeichnet, dass sie enthält :

— eine Mischung synthetischer Polymeren (Carboxypolymethylene) und modifizierter Cellulosen (Natrium-Carraghenat) : höchstens 3,5 % ;

— Kieselsäure : 8 bis 25 % ;

— Kieselerde : 1 bis 6 % ;

— Natrium-dodecylhydrogensulfat : 1,5 bis 3 % ;

— Propandiol : 3 bis 4 % ;

— Sorbitol : 20 bis 60 % ;

— Ester der p-Hydroxybenzoesäure : 0,1 bis 0,5 % ;

— Chlorphenolrot : 0,02 bis 0,1 % ;

— Aromen : etwa 1,5 % ;

— Lebensmittelfarbstoffe : 0,01 bis 0,04 % ;

— Zitrat-/Zitronensäure-Puffer mit einem solchen Ionendruck und pH-Wert, dass ein pH-Endwert zwischen 4,0 und 5,5 besteht : 10 bis 50 % ;

— Wasser bis 100 %.

3. Zahnpaste nach Anspruch 2, dadurch gekennzeichnet, dass das Carboxypolymethylen und das Carraghenat Carbopol 940 und Satiagum B sind.

4. Zahnpaste nach Anspruch 2, dadurch gekennzeichnet, dass die Ester der p-Hydroxybenzoesäure eine Mischung von p-Hydroxybenzoesäure-methylester und p-Hydroxybenzoesäure-propylester sind.

5. Zahnpaste nach Anspruch 2, dadurch gekennzeichnet, dass der Zitrat-Puffer aus Zitronensäure von 0,07 M und Natriumzitat von 0,07 M, eingestellt auf einen pH-Wert zwischen 4,2 und 4,6, besteht.

6. Zahnpaste nach Anspruch 2, dadurch gekennzeichnet, dass der Lebensmittelfarbstoff Mohnblumen-rot in geringer Konzentration (0,01 bis 0,04 %) ist, um das Auftreten des Farbumschlages vom Chlorphenolrot nach Purpur nicht zu überdecken.

7. Zahnpaste nach Anspruch 1, dadurch gekennzeichnet, dass sie als ein bevorzugtes Ausführungs-

beispiel enthält :

| | |
|---|---|
| — Carbopol 940 | 1,5 % |
| — Satiagum B | 1,5 % |
| — Kieselsäure | 10,0 % |
| — Titandioxyd | 4,0 % |
| — Natrium-laurylsulfat | 2,1 % |
| — Sorbitol 70 % | 33,0 % |
| — p-Hydroxybenzoesäure-methylester | 0,15 % |
| — p-Hydroxybenzoesäure-propylester | 0,15 % |
| — Chlorphenolrot | 0,05 % |
| — Aromen | 1,3 % |
| — Natrium-saccharin | 0,5 % |
| — Natrium-monofluorphosphat | 0,75 % |
| — Bromchlorophen | 0,15 % |
| — Puffer : Zitronensäure/Natriumzitrat von 0,07 M und pH 4,5 zu | 100,00 g |